# EUROPÄISCHE PATENTSCHRIFT

(11) **EP 1 107 739 B1**
(45) Veröffentlichungstag und Bekanntmachung des Hinweises auf die Patenterteilung: **02.04.2003**
(21) Anmeldenummer: 99946047.0
(22) Anmeldetag: 27.08.1999
(51) Int. Cl.: A61K 9/20, A61K 9/28

(54) **VERFAHREN ZUR HERSTELLUNG VON FESTEN DOSIERUNGSFORMEN**
METHOD FOR PRODUCING SOLID DOSING FORMS
PROCEDE DE PREPARATION DE FORMES POSOLOGIQUES SOLIDES

(30) Priorität: 28.08.1998 DE 19839276
(43) Veröffentlichungstag der Anmeldung: 20.06.2001
(73) Patentinhaber: KNOLL AKTIENGESELLSCHAFT, 67061 Ludwigshafen (DE)
(72) Erfinder: SPENGLER, Reinhard, D-67133 Maxdorf (DE); ROSENBERG, Jörg, D-67158 Ellerstadt (DE)
(74) Vertreter: Kinzebach, Werner, Dr.
(86) Internationale Anmeldenummer: EP9906314
(87) Internationale Veröffentlichungsnummer: WO00012068

(56) Entgegenhaltungen:
- EP-A- 0 590 963
- EP-A- 0 864 324
- WO-A-93/07859
- WO-A-96/19963
- DE-A- 4 446 467

## Beschreibung

Die vorliegende Erfindung betrifft ein Verfahren zur Herstellung von festen Dosierungsformen durch Bildung eines plastischen Gemisches aus mindestens einem polymeren Bindemittel, mindestens einem Wirkstoff und gegebenenfalls üblichen Additiven und Formgebung des plastischen Gemisches zu den festen Dosierungsformen mittels eines Formwerkzeugs.

Die Herstellung von festen Dosierungsformen durch Extrusion und anschließende Kalandrierung einer wirkstoffhaltigen Schmelze ist z.B. aus der DE-A-17 66 546 und der US-A-4,880,585 bekannt. Grundlage dieses Verfahrens ist die Einbettung eines Wirkstoffes in eine Schmelze aus einem Träger, z. B. Fettsubstanzen oder wasserlöslichen, thermoplastischen Polymeren. Die Schmelze wird dadurch erzeugt, dass die Mischung aus Wirkstoff, Polymer und gegebenenfalls weiteren Hilfsstoffen beispielsweise in einem Extruder aufgeschmolzen und als Schmelze in einem nachgeschaltetem Formkalander zu Tabletten geformt wird, die durch Abkühlen aushärten.

Feste Dosierungsformen enthalten neben der Trägersubstanz in der Regel einen oder mehrere Hilfsstoffe. Diese Hilfsstoffe sind zum einen oft unverzichtbar, um die Freisetzung des Wirkstoffes zu steuern, eine Zersetzung des Wirkstoffes durch Licht und/oder Oxidation zu verhindern, einen unangenehmen Geschmack des Wirkstoffes zu überdecken, die Dosierungsformen zur leichteren Identifizierung farbig zu gestalten usw. Zum anderen sind Hilfsstoffe meist erforderlich, um eine gute technische Verarbeitbarkeit der Dosierungsformen bei der Herstellung, bei Nachbearbeitungsschritten usw. zu gewährleisten.

Die erforderlichen Hilfsstoffe werden meist in die Tablettenmasse eingearbeitet, d. h. sie sind in den fertigen Dosierungsformen im Volumen der Dosierungsformen gleichmäßig verteilt. Die meisten der eingesetzten Hilfsstoffe entfalten ihre Wirkung aber nur an der Oberfläche der Dosierungsformen. Dies bedeutet, dass lediglich der in der äußersten Oberflächenschicht und wenige Mikrometer dahinter befindliche Anteil der Hilfsstoffe zur erwünschten Wirkung beiträgt. Um eine ausreichende Konzentration an der Oberfläche zu erhalten, müssen diese Hilfsstoffe daher in relativ hoher Konzentration in die Tablettenmasse einbezogen werden. Die Hauptmenge des Hilfsstoff im Tablettenvolumen bleibt wirkungslos, was die Herstellung der Tabletten unnötig verteuert. Es kann außerdem zu unerwünschten Wechselwirkungen des Hilfsstoffs mit dem Wirkstoff bzw. den matrixbildenden Stoffen kommen. Darüber hinaus ist die Freiheit bei der Formulierung der Tablettenzusammensetzung eingeschränkt, da nur miteinander kompatible Wirkstoffe und Hilfsstoffe verwendet werden können.

Es ist andererseits bekannt, Tabletten im letzten Produktionsschritt mit einer dünnen Schicht aus z.B. wasserlöslichen Polymeren zu überziehen. Auf diese Weise werden sogenannte Filmtabletten hergestellt. Falls ein Überzug über die durch Schmelzkalandrierung hergestellten Tabletten gewünscht war, musste dieser Überzug nach dem Abkühlen der Tabletten in einem eigenen Arbeitsgang aufgebracht werden. Dies erfolgte in konventioneller Weise, beispielsweise durch Aufsprühen in rotierenden Trommeln, nach dem Tauchrohrverfahren oder in der Wirbelschicht etc. Die konventionellen Verfahren zur Aufbringung von Coating-Schichten verlangen alle einen vergleichsweise extrem hohen Energieeinsatz, da die verwendeten Lösungsmittel aus den Sprühlösungen nach dem Aufsprühen auf die Tabletten schnell wieder entfernt werden müssen. Zudem dauert ein Coating-Prozess meist mehrere Stunden, da die Sprührate nicht beliebig hoch eingestellt werden kann.

Die WO 96/19963 beschreibt ein Verfahren zur Herstellung von umhüllten Tabletten durch Schmelzkalandrierung, bei dem die wirkstoffhaltige Schmelze zwischen zwei Folien aus dem Umhüllungsmaterial in die Kalanderformwalzen eingeführt wird. Dieses Verfahren ist allerdings nur für Hilfsstoffe geeignet, die leicht in Form einer Folie hergestellt werden können.

Die DE-A-44 46 467 beschreibt ein Verfahren zur Herstellung von linsenförmigen Tabletten durch Schmelzkalandrierung. Es wird in dieser Druckschrift darauf verwiesen, daß Formwalzen verwendet werden können, die mit einem Trennmittel versehen sind. Als Trennmittel ist beispielsweise ein Silikonlack geeignet. Dies läßt darauf schließen, daß die Formwalzen nur einmalig mit dem Trennmittel ausgekleidet werden und keine Übertragung des Trennmittels von den Formwalzen auf das zu tablettierende Gemisch erfolgt. Die EP-A 864 324, die erst nach dem Prioritätsdatum der vorliegenden Anmeldung veröffentlicht wurde, beschreibt ebenfalls, die Vertiefungen einer Formwalze mit einem Formtrennmittel zu überziehen.

Die EP-A 590 963 beschreibt ein Verfahren und eine Vorrichtung zur Herstellung von Tabletten. Eine angefeuchtete Paste wird in ein Formwerkzeug gefüllt. Um ein Haften der Paste am Pressstempel zu vermeiden, werden Ober- und Unterseite mit einem Pulver bestäubt.

Die WO 93/07859 ofenbart wirkstoffhaltige Pellets, die durch Schmelzsphäronisierung hergestellt werden. Während der Sphäronisierung können Antihaftmittel zugegeben werden.

Der vorliegenden Erfindung liegt daher die Aufgabe zugrunde, ein Verfahren zur Herstellung von festen Dosierungsformen durch Schmelzextrusion zur Verfügung zu stellen, bei dem eine Modifizierung der Oberflächeneigenschaften der festen Dosierungsformen auf einfache, kosten- und materialsparende Weise möglich ist.

Überraschenderweise wurde nun gefunden, dass diese Aufgabe gelöst wird, wenn bei der Formgebung ein Oberflächeneigenschaften-modifizierendes Mittel in feinverteilter Form auf die Oberfläche des plastischen Gemisches aufgebracht wird. Die durch die Formgebung zu festen Dosierungsformen geschaffene äußere Oberfläche des plastischen Gemisches wird auf diese Weise mit einem Oberflächeneigenschaften-modifizierenden Mittel versehen.

Gegenstand der vorliegenden Erfindung ist daher ein Verfahren zur Herstellung von festen Dosierungsformen durch Bildung eines plastischen Gemisches aus mindestens einem polymeren Bindemittel, mindestens einem Wirkstoff und gegebenenfalls üblichen Additiven und Formgebung des lösungsmittelfreien plastischen Gemisches zu den festen Dosierungsformen mittels eines Formwerkzeugs, das dadurch gekennzeichnet ist, dass bei der Formgebung ein Oberflächeneigenschaften-modifizierendes Mittel in feinverteilter Form auf die Oberfläche des plastischen Gemisches in einer Menge aufgebracht wird, dass das Oberflächeneigenschaften-modifizierende Mittel in der erhaltenen festen Dosierungsform 0,01 bis 1,0 Gew.-%, bezogen auf das Gesamtgewicht der festen Dosierungsform, ausmacht, wobei das Oberflächeneigenschaften-modifizierende Mittel zunächst in feinverteilter Form auf die Oberflächen des Formwerkzeugs aufgebracht wird, die mit dem zu formenden plastischen Gemisch in Kontakt kommen und nach jedem Kontakt der Oberflächen des Formwerkzeugs mit dem plastischen Gemisch ein neuer Auftrag von Oberflächeneigenschaften-modifizierendem Mittel erfolgt.

Das erfindungsgemäße Verfahren wird vorzugsweise kontinuierlich durchgeführt. Es ist für das erfindungsgemäße Verfahren wesentlich, dass für eine kontinuierliche Zufuhr an Oberflächeneigenschaften-modifizierendem Mittel gesorgt wird. Es ist außerdem wichtig, daß das Oberflächeneigenschaften-modifizierende Mittel zu einem Zeitpunkt aufgebracht wird, bei dem das plastische Gemisch noch nicht völlig erstarrt ist.

Der Begriff "Formgebung" kann die Schritte Vorformung und abschließende Formgebung umfassen. Unter "Vorformung" ist jede Maßnahme zu verstehen, bei der das Verhältnis der Oberfläche zum Volumen des plastischen Gemisches bereits weitgehend demjenigen der fertigen Dosierungsform angenähert wird. Vorzugsweise wird das vorgeformte plastische Gemisch bei der abschließenden Formgebung keinen wesentlichen Scherkräften ausgesetzt, so daß Oberflächenelemente nicht in das Innere eingemischt werden.

Als Vorformung wird z.B. die Extrusion des plastischen Gemisches zu einem Strang oder zu einer Breitware angesehen. So kann im Rahmen der vorliegenden Erfindung das Oberflächeneigenschaften-modifizierende Mittel auf die Oberfläche des vorgeformten plastischen Gemisches, z.B. des Stranges oder der Breitware, aufgebracht werden, das anschließend der abschließenden Formgebung unterzogen wird.

Das erfindungsgemäße Verfahren ermöglicht eine wesentliche Einsparung an Hilfsstoffen ohne Einbuße an Wirksamkeit, da die erfindungsgemäß erhaltenen Dosierungsformen die Hilfsstoffe überwiegend oder ausschließlich an ihrer Oberfläche enthalten, während die Konzentration der Hilfsstoffe über das Volumen der Dosierungsformen gesenkt werden kann bzw. die zu tablettierende Masse frei von Hilfsstoffen gehalten werden kann.

Das Oberflächeneigenschaften-modifizierende Mittel wird in feinverteilter Form aufgebracht. Das heißt, das Mittel liegt in pulverförmiger oder flüssiger Form und nicht in kompakter Form, z.B. als Folie, vor. Das Oberflächeneigenschaften-modifizierende Mittel kann pulverförmig, als Lösung, als Suspension, Emulsion oder Dispersion aufgebracht werden. Soweit zur Herstellung einer Lösung, Suspension, Emulsion oder Dispersion Trägerflüssigkeiten erforderlich sind, können diese verdunstend oder auch nichtverdunstend gewählt werden.

Unter Oberflächeneigenschaften-modifizierenden Mittel im Sinne der Erfindung werden alle pharmazeutisch verträglichen Hilfsstoffe verstanden, die die physikalischen und/oder chemischen Eigenschaften der Dosierungsformen, die ganz oder teilweise durch Oberflächeneffekte bedingt werden, z. B. Farbe, Schüttfähigkeit, Vereinzelungsverhalten, Gleitfähigkeit, Siebbarkeit, Durchlässigkeit für Dämpfe und/oder Gase, Lichtdurchlässigkeit, Lipophilie/Hydrophilie, Redoxpotential, Oberflächenspannung usw. verändern. Sie umfassen Hilfsstoffe, die üblicherweise in die Tablettenmasse eingearbeitet werden, und solche, deren Anwendung durch die vorliegende Erfindung erst ermöglicht wird.

Je nach Art des verwendeten Oberflächeneigenschaften-modifizierenden Mittels wird dieses in der Oberfläche des plastischen Gemisches eingebettet bzw. angelöst. In jedem Fall ist eine dauerhafte Verbindung des Oberflächeneigenschaften-modifizierenden Mittels mit den erhaltenen Dosierungsformen angestrebt.

In einer bevorzugten Ausführungsform der Erfindung handelt es sich bei dem Oberflächeneigenschaften-modifizierenden Mittel um ein Tensid, vorzugsweise eines HLB-Wertes von mehr als 10. Tenside können die Benetzbarkeit der Dosierungsformen erhöhen und somit die Löseeigenschaften verbessern. Daneben können Tenside die Entformbarkeit der Dosierungsformen verbessern. Als geeignete Tenside lassen sich anführen: Monofettsäureester des Polyhydroxyethylensorbitans, wie Polyethylenglycol(20)-sorbitan-monolaurat (HLB-Wert 16,7), Polyethylenglycol(20)-sorbitan-monostearat (HLB-Wert 14,9), Polyethylenglycol(20)-sorbitan-monooleat (HLB-Wert 15,0), Polyhydroxyethylen-fettalkoholether oder -fettsäureester, wie Polyhydroxyethylen-cetylstearylether (Cremophor®, HLB-Wert 16,1), Polyhydroxyethylen(23)-laurylether (HLB-Wert 16,9), Polyhydroxyethylen(8)-stearat (HLB-Wert 11,1), Polyhydroxyethylen(40)-stearat (HLB-Wert 16,8), Polyhydroxyethylen(100)-stearat (HLB-Wert 18,8), Ethylenoxid-Propylenoxid-Blockcopolymere (Pluronic®)' ethoxylierte Triglyceride, z. B. polyethoxyliertes Rizinusöl (40) (Cremophor®; HLB-Wert 12-14), polyethoxyliertes hydriertes Rizinusöl(40) (Cremophor®RH; HLB-Wert 14-16).

In einer weiteren bevorzugten Ausführungsform der Erfindung handelt es sich bei dem Oberflächeneigenschaften-modifizierenden Mittel um eine lipophile Verbindung, die vorzugsweise unter Glyceriden, Wachsen, Fettsäuren, Fettalkoholen, Paraffinen, Siliconen und Phosphatiden ausgewählt ist. Die Verwendung einer lipophilen Verbindung im Rahmen des erfindungsgemäßen Verfahrens gestattet eine Lipophilisierung der Oberfläche der Dosierungsformen. Damit kann z. B. die Freisetzung des Wirkstoffs und damit der Wirkungseintritt eines Arzneimittels zeitlich verzögert werden. Darüber hinaus kann das Verkleben von Dosierungsformen, die als Schüttung gelagert werden, vermindert werden. Das Eindringen von Wasser bzw. Wasserdampf oder anderen Bestandteilen der Luft in die Dosierungsform kann verringert oder unterbunden werden, was sich vorteilhaft auf die Lagerstabilität der Dosierungsformen auswirkt. Beispielhafte lipophile Verbindungen, die im Rahmen der Erfindung geeignet sind, sind u. a. Kohlenwasserstoffe, wie flüssiges und festes Paraffin, Vaseline; Fettalkohole, wie Cetylalkohol, Stearylalkohol, Cetostearylalkohol (Lanette®), 2-Octyl-dodecanol (Eutanol®); Fettsäuren, wie Stearinsäure; Salze von Fettsäuren, wie Magnesiumstearat, Calciumstearat; Glyceride, wie Erdnußöl, Olivenöl, Sesamöl, hydriertes Erdnußöl, hydriertes Baumwollsamenöl, hydriertes Rizinusöl, halbsynthetische und synthetische Glyceride; Wachse, wie Bienenwachs, Carnaubawachs, Cetylpalmitat, Wollwachs (Lanolin®), Isopropylmyristat, Isopropylstearat, Cetiol®, Ethyloleat; Phosphatide, wie Ei-Lecithin oder Soya-Lecithin.

In einer weiteren bevorzugten Ausführungsform der Erfindung handelt es sich bei dem Oberflächeneigenschaften-modifizierenden Mittel um ein Antioxidationsmittel, das vorzugsweise unter Ascorbinsäure, Ascorbylpalmitat, Butylhydroxytoluol, Butylhydroxyanisol, Propylgallat und Tocopherolen ausgewählt ist. Wachsartige Antioxidantien, insbesondere mit einem Schmelzpunkt von weniger als 100°C, sind bevorzugt. Der Einsatz eines Antioxidationsmittels verhindert, dass oxidationsempfindliche Inhaltsstoffe der Dosierungsformen während der Lagerung durch Zutritt von Luftsauerstoff oxidiert werden.

In einer weiteren bevorzugten Ausführungsform der Erfindung handelt es sich bei dem Oberflächeneigenschaften-modifizierenden Mittel um ein Farbmittel, z. B. einen löslichen Farbstoff oder auch ein anorganisches oder organisches Pigment. Als geeignete Farbmittel lassen sich Eisenoxide, Talkum, Calciumcarbonat, Titandioxid, z. B. mit Aluminiumoxid verlackte organische Farbstoffe, wie Erythrosin, Gelborange S, Tartrazin, Indigotin, Ponceau 4 R, Chinolingelb, Patentblau V; Azofarbstoffe, usw. anführen. Es hat sich gezeigt, dass die Verwendung unlöslicher Farbpigmente, wie z. B. Eisenoxiden, mit einer Verbesserung der Entformbarkeit verbunden ist.

Bei einer weiteren bevorzugten Ausführungsform der Erfindung handelt es sich bei dem Oberflächeneigenschaften-modifizierenden Mittel um ein Mittel mit antistatischer Wirkung oder/oder Gleitmittelwirkung. Antistatische Mittel verbessern die Schüttfähigkeit, das Vereinzelungsverhalten, die Gleitfähigkeit und die Siebbarkeit der Dosierungsformen. Als geeignete antistatische Mittel lassen sich u. a. Glykol, Glycerin, Aerosil, Polyethylenglykolester, Polyethylenglykol, Dicalciumphosphat und Lactose anführen. Gleitmittel dienen der Verbesserung der Entformung der Dosierungsformen aus dem Formwerkzeug. Als geeignete Gleitmittel lassen sich u. a. Magnesiumstearat, Calciumbehenat, Natriumstearylfumarat, Polyethylenglykole, Phosphatidylcholinderivate, Stearinsäure, Talkum, Aerosil, Calciumstearat, Glycerinester, hydriertes Baumwollsamenöl, hydriertes Rizinusöl und Reisstärke anführen.

Es versteht sich, dass ein Oberflächeneigenschaften-modifizierendes Mittel gleichzeitig mehreren der vorgenannten bevorzugten Ausführungsformen unterfallen kann. Andererseits können Oberflächeneigenschaften-modifizierende Mittel gemäß zweier oder mehrerer der vorgenannten Ausführungsformen kombiniert werden und gleichzeitig oder nacheinander aufgebracht werden.

Das Oberflächeneigenschaften-modifizierende Mittel bzw. das Gemisch Oberflächeneigenschaftenmodifizierender Mittel macht in der erhaltenen festen Dosierungsform 0,01 bis 1,0 Gew.-%, vorzugsweise 0,01 bis 0,5 Gew.-%, insbesondere 0,01 bis 0,3 Gew.-%, bezogen auf das Gesamtgewicht der festen Dosierungsform, aus.

Unter Dosierungsformen sind vorliegend alle Formen zu verstehen, die zur Verwendung als Arzneimittel, Pflanzenbehandlungsmittel, Futtermittel und Nahrungsmittel und zur Abgabe von Riechstoffen und Parfümölen geeignet sind. Dazu gehören beispielsweise Tabletten jeglicher Form, Pellets, Granulate, aber auch größere Formen, wie Würfel, Blöcke (Quader) oder zylindrische Formen, die sich insbesondere als Futter- oder Nahrungsmittel verwenden lassen.

Das plastische Gemisch umfasst im Allgemeinen:
a) 0,1 bis 90 Gew.-%, insbesondere 0,1 bis 60 Gew.-% (bezogen auf das Gesamtgewicht der Dosierungsform) eines Wirkstoffes,
b) 10 bis 99,9 Gew.-%, insbesondere 40 bis 99,9 Gew.-% eines polymeren Bindemittels, und
c) gegebenenfalls Additive.

Das Bindemittel soll vorzugsweise in physiologischer Umgebung löslich oder quellbar sein. Beispiele für geeignete Bindemittel sind Polyvinylpyrrolidon (PVP), Copolymerisate von N-Vinylpyrrolidon (NVP) und Vinylestern, insbesondere Vinylacetat, Copolymerisate von Vinylacetat und Crotonsäure, teilverseiftes Polyvinylacetat, Polyvinylalkohol, Polyhydroxyalkylacrylate, Polyhydroxyalkylmethacrylate, Polyacrylate und Polymethacrylate (Eudragit-Typen), Copolymerisate von Methylmethacrylat und Acrylsäure, Celluloseester, Celluloseether, insbesondere Methylcellulose und Ethylcellulose, Hydroxyalkylcellulosen, insbesondere Hydroxypropylcellulose, Hydroxyalkyl-Alkylcellulosen, insbesondere Hydroxypropylethylcellulose, Cellulosephthalate, insbesondere Celluloseacetatphthalat und Hydroxypropylmethylcellulosephthalat, Stärke, Stärkederivate, z. B. Maltodextrine, Zuckeralkohole, wie Mannit oder Palatinose und Mannane, insbesondere Galactomannane. Die K-Werte (nach H. Fikentscher, Cellulose-Chemie 13, 1932, S. 58-64 und 71 u. 74) der Polymeren liegen im Bereich von 10 bis 100, vorzugsweise 12 bis 70, insbesondere 12 bis 35, für PVP vorzugsweise bei 12 bis 35, insbesondere bei 12 bis 17.

Das polymere Bindemittel muss in der Gesamtmischung aller Komponenten im Bereich von 50 bis 180, vorzugsweise 60 bis 130 °C erweichen oder schmelzen, so dass die Masse extrudierbar ist. Die Glasübergangstemperatur der Mischung muss daher unter 180 °C, vorzugsweise unter 130 °C liegen. Erforderlichenfalls wird sie durch übliche pharmakologisch akzeptable, weichmachende Hilfsstoffe herabgesetzt, wie langkettige Alkohole, Ethylenglykol, Propylenglykol, Glycerin, Trimethylolpropan, Triethylenglykol, Zuckeralkohole, z. B. Butandiole, Pentanole, sowie Pentaerythrit oder Hexanole, Polyethylenglykole, Polypropylenglykole, Polyethylen-Propylenglykole, Silicone, aromatische Carbonsäureester (z. B. Dialkylphthalate, Trimellithsäureester, Benzoesäureester, Terephthalsäureester) oder aliphatische Dicarbonsäureester (z. B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt im Allgemeinen 0,5 bis 15 Gew.-%, vorzugsweise 0,5 bis 5 Gew.-%, bezogen auf das Gesamtgewicht der Masse. Vorzugsweise umfasst die Mischung keinen Weichmacher. Unter pharmazeutischen Wirkstoffen im Sinne der Erfindung sind alle Stoffe mit einer pharmazeutischen Wirkung und möglichst geringen Nebenwirkungen zu verstehen, sofern sie sich unter den Verarbeitungsbedingungen nicht zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, daß sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.-% liegen. Auch Wirkstoff-Kombinationen, z.B. Ibuprofen/Coffein, können eingesetzt werden. Wirkstoffe im Sinne der Erfindung sind auch Vitamine und Mineralstoffe, sowie Pflanzenbehandlungsmittel und Insektizide. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z.B. Adenin, Cholin, Pantothensäure, Biotin, Adenylsäure, Folsäure, Orotsäure, Pangamsäure, Carnitin, p-Aminobenzoesäure, myo-Inosit und Liponsäure, sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika.

Unter Wirkstoffen im Sinne der Erfindung sind alle Stoffe mit einer physiologischen Wirkung zu verstehen, sofern sie sich unter den Verarbeitungsbedingungen nicht zersetzen. Es handelt sich insbesondere um pharmazeutische Wirkstoffe (für Mensch und Tier), Wirkstoffe für die Pflanzenbehandlung, Insektizide, Futter- und Nahrungsmittelwirkstoffe, Riechstoffe und Parfümöle. Die Wirkstoffmenge pro Dosiseinheit und die Konzentration können je nach Wirksamkeit und Freisetzungsgeschwindigkeit in weiten Grenzen variieren. Die einzige Bedingung ist, dass sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.-% liegen. Auch Wirkstoff-Kombinationen können eingesetzt werden. Wirkstoffe im Sinne der Erfindung sind auch Vitamine und Mineralstoffe. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika. Zu Pflanzenbehandlungsmitteln zählen z. B. Vinclozolin, Epoxiconazol und Quinmerac.

Das erfindungsgemäße Verfahren ist beispielsweise zur Verarbeitung folgender Wirkstoffe geeignet:

Acebutolol, Acetylcystein, Acetylsalicylsäure, Acyclovir, Alprazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefadroxil, Cefazolin, Cefixim, Cefotaxim, Ceftazidim, Ceftriaxon, Cefuroxim, Selegilin, Chloramphenicol, Chlorhexidin, Chlorpheniramin, Chlortalidon, Cholin, Cyclosporin, Cilastatin, Cimetidin, Ciprofloxacin, Cisapride, Cisplatin, Clarithromycin, Clävulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Ginkgo Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Indomethacin, Iohexol, Iopamidol, Isosorbid-Dinitrat, Isosorbid-Mononitrat, Isotretinoin, Ketotifen, Ketoconazol, Ketoprofen, Ketorolac, Labetalol, Lactulose, Lecithin, Levocarnitin, Levodopa, Levoglutamide, Levonorgestrel, Levothyroxin, Lidocain, Lipase, Imipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Pentoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Bromocriptin, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Folinsäure, Zidovudin.

Bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin oder Captopril.

Neben dem zwingend vorhandenen Oberflächeneigenschaften-modifizierenden Mittel können die erfindungsgemäß erhaltenen Dosierungsformen in der Matrix übliche galenische Hilfsstoffe, deren Gesamtmenge bis zu 100 Gew.-%, bezogen auf das polymere Bindemittel, betragen kann, enthalten. Übliche galenische Hilfsstoffe sind z. B. Streckmittel bzw. Füllstoffe wie Magnesiumoxid, Aluminiumoxid, Titanoxid, Stearinsäure oder deren Salze, z. B. das Magnesium- oder Calciumsalz, Methylcellulose, Natrium-Carboxymethylcellulose, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht des Gemisches.

Schmiermittel, wie Aluminium- und Calciumstearat, Talkum und Silicone, in einer Konzentration von 0,1 bis 5, vorzugsweise 0,1 bis 3 Gew.-%, bezogen auf das Gesamtgewicht des Gemisches.

Fließmittel, wie tierische oder pflanzliche Fette, insbesondere in hydrierter Form und solche, die bei Raumtemperatur fest sind. Diese Fette haben vorzugsweise einen Schmelzpunkt von 50 °C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. Die Gesamtmenge an Fetten, Wachsen, Mono-, Diglyceriden und/oder Lecithinen beträgt 0,1 bis 30, vorzugsweise 0,1 bis 5 Gew.-%, bezogen auf das Gesamtgewicht der Masse für die jeweilige Schicht; Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind;

Stabilisatoren, wie Antioxidanzien, Lichtstabilisatoren, Hydroperoxid-Vernichter, Radikalfänger, Stabilisatoren gegen mikrobiellen Befall.

Ferner können Netz-, Konservierungs-, Spreng-, Adsorptions-, Formentrenn- und Treibmittel zugesetzt werden (vgl. z. B. H. Sucker et al., Pharmazeutische Technologie, Thieme-Verlag, Stuttgart 1978).

Unter Hilfsstoffen im Sinne der Erfindung sind auch Substanzen zur Herstellung einer festen Lösung des Wirkstoffs zu verstehen. Diese Hilfsstoffe sind beispielsweise Pentaerythrit und Pentaerythrit-tetraacetat, Polymere wie z. B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere, wie z. B. bei J. L. Ford, Pharm. Acta Helv. 61, 69-88 (1986) angegeben.

Als Hilfsstoffe gelten auch Zusätze von Basen und Säuren zur Steuerung der Löslichkeit eines Wirkstoffes (siehe beispielsweise K. Thoma et al., Pharm. Ind. 51, 98-101 (1989)).

Einzige Voraussetzung für die Eignung von Hilfsstoffen ist eine ausreichende Temperaturstabilität.

Zur Herstellung der festen Dosierungsformen wird ein plastisches Gemisch der Komponenten (Schmelze) bereitgestellt, das anschließend einem Formgebungsschnitt unterzogen wird. Das Vermischen der Komponenten und die Bildung der Schmelze können auf unterschiedliche Weise erfolgen. Das Vermischen kann vor, während und/oder nach der Bildung der Schmelze erfolgen. Beispielsweise können die Komponenten zuerst vermischt und dann aufgeschmolzen oder gleichzeitig vermischt und aufgeschmolzen werden. Häufig erfolgt noch eine Homogenisierung des plastischen Gemisches, um eine hochdisperse Verteilung des Wirkstoffes zu erhalten.

Insbesondere bei Verwendung von empfindlichen wirkstoffen hat es sich aber als bevorzugt erwiesen, zuerst das polymere Bindemittel, gegebenenfalls zusammen mit üblichen pharmazeutischen Additiven, aufzuschmelzen und vorzuvermischen und dann den (die) empfindlichen Wirkstoff(e) in "Intensivmischern" in plastischer Phase bei sehr kleinen Verweilzeiten einzumischen (Homogenisieren). Der (die) Wirkstoff(e) kann (können) dabei in fester Form oder als Lösung oder Dispersion eingesetzt werden.

Im Allgemeinen werden die Komponenten als solche in das Herstellungsverfahren eingesetzt. Sie können jedoch auch in flüssiger Form, d. h. als Lösung, Suspension oder Dispersion zur Anwendung kommen.

Als Lösungsmittel für die flüssige Form der Komponenten kommt in erster Linie Wasser oder ein mit Wasser mischbares, organisches Lösungsmittel oder ein Gemisch davon mit Wasser in Betracht. Brauchbare Lösungsmittel sind aber auch mit Wasser nicht mischbare oder mischbare, organische Lösungsmittel. Geeignete, mit Wasser mischbare Lösungsmittel sind insbesondere Aceton, C₁-C₄-Alkanole, wie Ethanol, Isopropanol oder n-Propanol, Polyole, wie Ethylenglykol, Glycerin und Polyethylenglykole. Geeignete, mit Wasser nicht mischbare Lösungsmittel sind Alkane, wie Pentan oder Hexan, Ester, wie Ethylacetat oder Butylacetat, chlorierte Kohlenwasserstoffe, wie Methylenchlorid und aromatische Kohlenwasserstoffe, wie Toluol und Xylol. Ein weiteres brauchbares Lösungsmittel ist flüssiges CO₂.

Welches Lösungsmittel im Einzelfall verwendet wird, hängt von der aufzunehmenden Komponente und deren Eigenschaften ab. Beispielsweise kommen pharmazeutische Wirkstoffe häufig in Form eines Salzes, das im Allgemeinen wasserlöslich ist, zur Anwendung. Wasserlösliche Wirkstoffe können daher als wässrige Lösung eingesetzt werden oder vorzugsweise in die wässrige Lösung oder Dispersion des Bindemittels aufgenommen werden. Entsprechendes gilt für Wirkstoffe, die in einem der genannten Lösungsmittel löslich sind, wenn die flüssige Form der zur Anwendung kommenden Komponenten auf einem organischen Lösungsmittel basiert.

Gegebenenfalls kann an die Stelle des Aufschmelzens ein Lösen, Suspendieren oder Dispergieren in den oben genannten Lösungsmitteln, falls erwünscht und/oder erforderlich unter Zusatz geeigneter Hilfsstoffe, wie z. B. Emulgatoren, treten. Das Lösungsmittel wird dann im Allgemeinen unter Bildung der Schmelze in einer geeigneten Apparatur, z. B. einem Extruder, entfernt. Im Folgenden soll dies von dem Begriff Vermischen umfasst werden.

Das Aufschmelzen und/oder Vermischen erfolgt in einer für diesen Zweck üblichen Vorrichtung. Besonders geeignet sind Extruder oder gegebenenfalls beheizbare Behälter mit Rührwerk, z. B. Kneter, (wie der unten noch erwähnten Art).

Als Mischapparat sind insbesondere solche Vorrichtungen brauchbar, die in der Kunststofftechnologie zum Mischen eingesetzt werden. Geeignete Vorrichtungen sind beispielsweise beschrieben in "Mischen beim Herstellen und Verarbeiten von Kunststoffen", H. Pahl, VDI-Verlag, 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise Misch-Knetreaktoren (z. B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z. B. Dispax der Firma IKA).

Bei empfindlichen Wirkstoffen erfolgt vorzugsweise zunächst das Aufschmelzen des polymeren Bindemittels in einem Extruder und anschließend das Zumischen des Wirkstoffs in einem Misch-Knetreaktor. Bei weniger empfindlichen Wirkstoffen kann man dagegen zum intensiven Dispergieren des Wirkstoffs ein Rotor/Stator-System einsetzen.

Das Beschicken der Mischvorrichtung erfolgt je nach deren Konzeption kontinuierlich oder diskontinuierlich in üblicher Weise. Pulverförmige Komponenten können im freien Zulauf, z. B. über eine Differentialdosierwaage eingeführt werden. Plastische Massen können direkt aus einem Extruder eingespeist oder über eine Zahnradpumpe, die insbesondere bei hohen Viskositäten und hohen Drüken von Vorteil ist, zugespeist werden. Flüssige Medien können über ein geeignetes Pumpenaggregat zudosiert werden.

Das durch Vermischen und/oder Aufschmelzen des Bindemittels, des Wirkstoffes und gegebenenfalls des Additivs oder der Additive erhaltene Gemisch ist teigig bis zähflüssig (thermoplastisch) oder flüssig und daher extrudierbar. Die Glasübergangstemperatur des Gemisches liegt unter der Zersetzungstemperatur aller in dem Gemisch enthaltenen Komponenten. Das Bindemittel soll vorzugsweise in physiologischer Umgebung löslich oder quellbar sein.

Die Verfahrensschritte Vermischen und Aufschmelzen können in derselben Apparatur oder in zwei oder mehreren getrennt arbeitenden Vorrichtungen ausgeführt werden. Die Zubereitung einer Vormischung kann in einer der oben beschriebenen üblichen Mischvorrichtungen durchgeführt werden. Eine solche Vormischung kann dann direkt, z. B. in einen Extruder, eingespeist und anschließend ggf. unter Zusatz weiterer Komponenten extrudiert werden.

Das erfindungsgemäße Verfahren erlaubt es, als Extruder Einschneckenmaschinen, kämmende Schneckenmaschinen oder auch Mehrwellen-extruder, insbesondere Zweischnecken-Extruder, gleichsinnig oder gegensinnig drehend und gegebenenfalls mit Knetscheiben ausgerüstet, einzusetzen. Wenn bei der Extrusion ein Lösungsmittel verdampft werden muss, sind die Extruder im Allgemeinen mit einem Verdampfungsteil ausgerüstet. Besonders bevorzugt sind Extruder der ZKS-Baureihe von Werner u. Pfleiderer.

Das erhaltene Gemisch ist lösungsmittelfrei, d. h. es enthält weder Wasser noch ein organisches Lösungsmittel. Der extrudierte, verformbare Strang des plastischen Gemisches wird einer abschließenden Formgebung zu den festen Dosierungsformen mittels eines Formwerkzeugs unterzogen. Dabei kann eine Vielzahl von Formen, je nach Werkzeug und Art der Formung, erzeugt werden. Beispielsweise lässt sich bei Verwendung eines Extruders der extrudierte Strang zwischen einem Band und einer Walze, zwischen zwei Bändern oder zwischen zwei Walzen, wie in der EP-A-358 105 beschrieben, formen. Besonders bevorzugt ist die Verwendung eines Formwerkzeugs mit zwei gegenläufig rotierenden Formwalzen, von denen wenigstens eine Vertiefungen zur Aufnahme und Formung des plastischen Gemisches aufweist. Diese Art der Formgebung wird üblicherweise als Kalandrierung bezeichnet und ist beispielsweise in der EP-A-240 904 beschrieben. Durch Extrusion und Heiß- oder Kaltabschlag des Stranges können weitere Formen erhalten werden, beispielsweise kleinteilige und gleichmäßig geformte Granulate. Die Heißgranulierung führt in der Regel zu linsenförmigen Dosierungsformen (Tabletten) mit einem Durchmesser von 1 bis 10 mm, während die Kaltgranulierung normalerweise zu zylinderförmigen Produkten mit einem Verhältnis von Länge zu Durchmesser von 1 bis 10 mm und einem Durchmesser von 0,5 bis 10 mm führt. So können beispielsweise Oblong-Tabletten, Dragees, Pastillen und Pellets hergestellt werden.

Beim erfindungsgemäßen Verfahren wird das Oberflächeneigenschaften-modifizierende Mittel zunächst in feinverteilter Form auf die Oberflächen des Formwerkzeugs aufgebracht, die mit dem plastischen Gemisch in Kontakt kommen. Nach jedem Kontakt der Oberflächen des Formwerkzeuges mit dem plastischen Gemisch erfolgt ein neuer Auftrag von Oberflächeneigenschaften-modifizierendem Mittel. So können bei Verwendung eines Formwerkzeugs mit zwei gegenläufig rotierenden Formwalzen, von denen wenigstens eine Vertiefungen zur Aufnahme und Formung des plastischen Gemisches aufweist (Formkalander), die Formwalzen mit dem Oberflächeneigenschaften-modifizierenden Mittel versehen werden. Sobald das plastische Gemisch in die Vertiefungen eintritt, nimmt die weiche, bisweilen klebrige Oberfläche des plastischen Gemisches das Oberflächeneigenschaften-modifizierende Mittel auf und es entsteht eine dauerhafte Verbindung zwischen beiden. Der Auftrag des Oberflächeneigenschaften-modifizierenden Mittels auf die Formwalzen kann z.B. erfolgen, indem die Walzen an ihrer Unterseite durch ein Bad laufen, indem das Mittel aufgesprüht oder mittels Pinseln oder Bürsten, z.B. mittels eines Bürstenkranzes mit Schlauchanschluß, aufgebracht wird.

Besonders gute Ergebnisse wurden unter Verwendung der folgenden Oberflächeneigenschaften-modifizierenden Mittel in der angegebenen Konzentration (Gew.-%, bezogen auf die Dosierungseinheit) erhalten: Magnesiumstearat (0,01 bis 0,3 %), Calciumstearat (0,01 bis 0,3 %), Calciumbehenat (0,01 bis 0,3 %), Natriumstearylfumarat (0,01 bis 1,0 %), Lecithin (z. B. Ei/Soja) (0,01 bis 0,5 %), polyethylenglykol (0,01 bis 1,0 %), Stearinsäure, (0,01 bis 1,0 %), hydriertes Baumwollsamenöl (0,01 bis 0,3 %), hydriertes Rizinusöl (0,01 bis 0,3 %), Talkum (0,01 bis 2,0 %), Reisstärke (0,01 bis 2,0 %), Calciumcarbonat (0,01 bis 1,0 %), Titandioxid (0,01 bis 2,0 %), hochdisperses Siliciumdioxid (0,01 bis 0,1 %), verlackte Farbstoffe (0,01 bis 0,5 %), Eisenoxidfarbstoffe (0,01 bis 0,5 %), Silicone (0,01 bis 0,5 %), Paraffine (0,01 bis 0,5 %), Carnaubawachs (0,01 bis 0,5 %), Bienenwachs (0,01 bis 0,5 %), Ethyloleat (0,01 bis 0,3 %), Stearylalkohol (0,01 bis 1,0 %), Tenside, z. B. Cremophor EL (0,01 bis 0,3 %).

Flüssige Oberflächeneigenschaften-modifizierende Mittel, wie z. B. Polyethylenglycole, Silicone, Paraffine, Ethyloleat, können direkt durch Eintauchen des vorgeformten plastischen Gemisches oder der Formwerkzeugoberflächen oder Aufpinseln oder Besprühen aufgebracht werden. Stoffe, die in einem Lösungsmittel gelöst werden können, wie z. B. Lecithin in Wasser oder Alkohol, Polyethylenglycol (mit einem Molekulargewicht von mehr als 1000) in Wasser, Tenside in Wasser oder z. B. Alkohol, werden als Lösung wie vorstehend beschrieben aufgebracht. Stoffe, die nicht oder nur unzureichend in einem Lösungsmittel aufgelöst werden können, wie z. B. Magnesiumstearat, Talkum oder Wachse, werden als Suspension oder Dispersion in einem Lösungsmittel, gegebenenfalls in Kombination mit flüssigen oder gelösten Oberflächeneigenschaften-modifizierenden Mitteln, wie vorstehend beschrieben aufgebracht. Stoffe, die nicht oder nur unzureichend aufgelöst werden können und zusätzlich sehr feinteilig vorliegen (Teilchengrößen von weniger als etwa 50 µm), können über eine Luftsprühvorrichtung (Zweistoff-Düsen) aufgesprüht werden. Zu diesen Stoffen gehören 2. B. Magnesiumstearat, Calciumcarbonat, Natriumstearylfumarat, Titandioxid und verlackte Farbstoffe. Das Aufsprühen kann je nach gewünschter Auftragsmenge trocken (ohne weiteren Hilfsstoff) oder suspendiert in einem Träger, wie Wasser oder Polyethylenglycol, oder in einem rasch verdunstenden Lösungsmittel, z. B. Aceton, erfolgen. Die genannten Methoden sind vorzugsweise dazu geeignet, einen Produktstrang kurz vor der Kalandrierung oder die Formwerkzeugoberflächen, die mit dem plastischen Gemisch in Kontakt kommen, mit einem Oberflächeneigenschaften-modifizierenden Mittel zu versehen.

Das erfindungsgemäße Verfahren weist folgende Vorteile auf: Die erfindungsgemäß erhaltenen festen Dosierungsformen enthalten das Oberflächeneigenschaften-modifizierende Mittel lediglich auf ihrer Oberfläche, nicht über die gesamte Masse. Dadurch lässt sich die Menge an Oberflächeneigenschaften-modifizierenden Mitteln erheblich verringern. Dies gestattet wiederum, die Dosierungsformen bei gleicher Wirkstoffmenge erheblich kleiner zu gestalten, was zu einer größeren Ausbeute pro Charge führt und das Herstellungsverfahren kostengünstiger macht. Beim erfindungsgemäßen Verfahren können auch Oberflächeneigenschaften-modifizierende Mittel verwendet werden, die mit einem oder mehreren Bestandteilen der Tablettenmasse inkompatibel sind, da das Oberflächeneigenschaften-modifizierende Mittel weitgehend räumlich von der Matrix der Dosierungsformen getrennt ist. Es kann eine Charge einer pharmazeutischen Mischung auf Vorrat hergestellt werden, die je nach Bedarf mit unterschiedlichen Oberflächeneigenschaften-modifizierenden Mitteln versehen werden kann. Insbesondere beim Einsatz von Oberflächeneigenschaften-modifizierenden Mitteln mit Gleitmittelwirkung sind Probleme im Zusammenhang mit der Klebrigkeit des plastischen Gemisches vermindert. Die geformten Dosierungsformen müssen beim Verlassen des Formwerkzeugs noch nicht völlig erkaltet sein und die Produktionsgeschwindigkeit lässt sich ohne Nachteil erhöhen.

### Beispiel

Es wurden Extrusionstabletten eines Vitamin B-Komplexes gemäß folgender Zusammensetzung hergestellt:

| | |
|---|---|
| Vitamin B-Komplex | 13,32 % |
| Klucel EF | 20,00 % |
| Isomalt | 66,68 % |

Es wurden linsenförmige Tabletten mit einer Masse von 250,0 mg hergestellt.

Zur Herstellung wurden der Vitamin B-Komplex, Klucel EF und Isomalt in einem Containermischer etwa 20 min lang gemischt und anschließend extrudiert. Die Einstellparameter der Extrusion sowie die Kalandertemperatur bei den Versuchen V 1 bis V 3 sind nachstehend angegeben:

| Parameter | V 1 | V 2 | V 3 |
|---|---|---|---|
| Massenfluß [kg/h] | 25 | 25 | 25 |
| Schneckendrehzahl [Upm] | 130 | 130 | 130 |
| Vakuum [mbar] | 150 | 105 | 105 |
| Temp. Einzug [°C] | 23 | 23 | 23 |
| Temp. Schuß 1 [°C] | 80 | 80 | 80 |
| Temp. Schuß 2 [°C] | 100 | 100 | 100 |
| Temp. Schuß 3 [°C] | 110 | 110 | 110 |
| Temp. Schuß 4 [°C] | 110 | 110 | 110 |
| Temp. Kopf [°C] | 120 | 120 | 120 |
| Temp. Düse [°C] | 120 | 120 | 125 |
| Temp. Kalander [°C] | 18 | 12 | 30 |

Die Tabletten ließen sich aufgrund einer starken Klebewirkung nicht aus den Formwalzen ablösen. In einem weiteren Versuch (V 4) wurde wie in Versuch V 3 verfahren, jedoch war unter jeder der beiden Kalanderwalzen eine Schale angebracht, die mit Ethyloleat gefüllt war. Die Eintauchtiefe der Walzen in die Flüssigkeit war so bemessen, dass alle Gravuren, die am unteren Scheitelpunkt der Walzen zu liegen kommen, benetzt wurden (minimale Eintauchtiefe). Die vorstehend beschriebene Tablettenmasse war aus den auf diese Weise benetzten Formwalzen praktisch ohne Klebewirkung entformbar.

Die durch Wägung ermittelte Massenzunahme der Tabletten durch das Ethyloleat betrug in diesem Fall etwa 0,1%.

In weiteren Versuchen wurden Kombinationen von Oberflächeneigenschaften-modifizierenden Mitteln eingesetzt, wie z. B. eine Suspension von 2% Magnesiumstearat in einer Mischung von 10% Polyethylenglycol 600 in Wasser. Auf der extrudierten Tablette verblieben dann etwa 0,15% Polyethylenglycol und etwa 0,03% Magnesiumstearat.

## Patentansprüche

1. Verfahren zur Herstellung von festen Dosierungsformen durch Bildung eines plastischen Gemisches aus 10 bis 99,9 Gew.-% mindestens eines polymeren Bindemittels, 0,1 bis 90 Gew.-% mindestens eines Wirkstoffs und gegebenenfalls üblichen Additiven und Formgebung des lösungsmittelfreien plastischen Gemisches zu den festen Dosierungsformen mittels eines Formwerkzeugs, **dadurch gekennzeichnet, dass** bei der Formgebung ein Oberflächeneigenschaften-modifizierendes Mittel in feinverteilter Form auf die Oberfläche des plastischen Gemisches in einer Menge aufgebracht wird, dass das Oberflächeneigenschaften-modifizierende Mittel in der erhaltenen festen Dosierungsform 0,01 bis 1,0 Gew.-%, bezogen auf das Gesamtgewicht der festen Dosierungsform, ausmacht, wobei das Oberflächeneigenschaften-modifizierende Mittel zunächst in feinverteilter Form auf die Oberflächen des Formwerkzeugs aufgebracht wird, die mit den zu formenden plastischen Gemisch in Kontakt kommen und nach jedem Kontakt der Oberflächen, des Formwerkzeugs mit dem plastischen Gemisch ein neuer Auftrag von Oberflächeneigenschaften-modifizierendem Mittel erfolgt.

2. verfahren nach Anspruch 1, **dadurch gekennzeichnet, dass** das Formwerkzeug zwei gegenläufig rotierende Formwalzen, von denen wenigstens eine Vertiefungen zur Aufnahme und Formung des plastischen Gemisches aufweist, umfasst.

3. Verfahren nach Anspruch 2, **dadurch gekennzeichnet, dass** der Auftrag des Oberflächeneigenschaften-modifizierenden Mittels auf die Formwalzen erfolgt, indem die Walzen an ihrer Unterseite durch ein Bad laufen, indem das Mittel aufgesprüht oder mittels Pinseln oder Bürsten aufgebracht wird.

4. Verfahren nach einem der vorhergehenden Ansprüche, **dadurch gekennzeichnet**, das es sich bei dem Oberflächeneigenschaften-modifizierenden Mittel um ein Tensid handelt.

5. Verfahren nach einem der Ansprüche 1 bis 3, **dadurch gekennzeichnet, dass** es sich bei dem Oberflächeneigenschaften-modifizierenden Mittel um eine lipophile Verbindung handelt, die ausgewählt ist unter Glyceriden, Wachsen, Fettsäuren, Fettalkoholen, Paraffinen, Siliconen und Phosphatiden.

6. Verfahren nach einem der Ansprüche 1 bis 3, **dadurch gekennzeichnet, dass** es sich bei dem Oberflächeneigenschaften-modifizierenden Mittel um ein Antioxidationsmittel handelt.

7. verfahren nach einem der Ansprüche 1 bis 3, **dadurch gekennzeichnet, dass** es sich bei dem Oberflächeneigenschaften-modifizierenden Mittel um ein Farbmittel handelt.

8. Verfahren nach einem der Ansprüche 1 bis 3, **dadurch gekennzeichnet, dass** es sich bei dem Oberflächeneigenschften-modifizierenden Mittel um ein Mittel mit antistatischer Wirkung und/oder Gleitmittelwirkung handelt.

## Claims

1. Process for producing solid dosage forms by forming a plastic mixture of from 10 to 99.9% by weight of at least one polymeric binder, from 0.1 to 90% by weight of at least one active ingredient and, where appropriate, conventional additives, and shaping the solvent-free plastic mixture to the solid dosage forms using a mold, **characterized in that** an agent which modifies the surface properties is applied in finely divided form to the surface of the plastic mixture during the shaping in an amount such that the agent which modifies the surface properties comprises from 0.01 to 1.0% by weight, based on the total weight of the resulting solid dosage form, where the agent which modifies the surface properties is initially applied in finely divided form to the surfaces of the mold which come into contact with the plastic mixture to be shaped, and a new application of the agent which modifies the surface properties takes place after each contact of the surfaces of the mold with the plastic mixture.

2. Process according to Claim 1, **characterized in that** the mold comprises two counterrotating molding rolls of which at least one has depressions to receive and shape the plastic mixture.

3. Process according to Claim 2, **characterized in that** the agent which modifies the surface properties is applied to the molding rolls by the underside of the rolls passing through a bath, by the agent being sprayed on or applied using fine or coarse brushes.

4. Process according to any of the preceding claims, **characterized in that** the agent which modifies the surface properties is a surfactant.

5. Process according to any of Claims 1 to 3, **characterized in that** the agent which modifies the surface properties is a lipophilic compound which is selected from glycerides, waxes, fatty acids, fatty alcohols, paraffins, silicones and phosphatides.

6. Process according to any of Claims 1 to 3, **characterized in that** the agent which modifies the surface properties is an antioxidant.

7. Process according to any of Claims 1 to 3, **characterized in that** the agent which modifies the surface properties is a coloring agent.

8. Process according to any of Claims 1 to 3, **characterized in that** the agent which modifies the surface properties is an agent with antistatic effect and/or lubricant effect.

## Revendications

1. Procédé pour la préparation de formes de dosage solides par formation d'un mélange plastique de 10 à 99,9 % en poids d'au moins un liant polymère, 0,1 à 90 % en poids d'au moins une substance active et le cas échéant des additifs usuels et façonnage du mélange plastique exempt de solvant en les formes de dosage solides à l'aide d'un outil approprié, **caractérisé par le fait que**, au façonnage, on applique sur la surface du mélange plastique un agent modifiant les propriétés de surface à l'état de fine division en quantité telle que dans la forme de dosage solide obtenue, l'agent modifiant les propriétés de surface représente de 0,01 à 1,0 % du poids total de la forme de dosage solide, l'agent modifiant les propriétés de surface étant d'abord appliqué à l'état de fine division sur les surfaces de l'outil de façonnage entrant en contact avec le mélange plastique à façonner, et, après chaque contact des surfaces de l'outil de façonnage avec le mélange plastique, on procède à une nouvelle application de l'agent modifiant les propriétés de surfaces.

2. Procédé selon la revendication 1, **caractérisé par le fait que** l'outil de façonnage comprend deux cylindres de façonnage tournant en sens contraire dont l'un au moins présente des cavités permettant de recevoir et de façonner le mélange plastique.

3. Procédé selon la revendication 2, **caractérisé par le fait que**, pour l'application de l'agent modifiant les propriétés de surface sur les cylindres de façonnage, la face inférieure des cylindres passe au travers d'un bain dans lequel le produit est appliqué par pulvérisation ou à l'aide de pinceaux ou de brosses.

4. Procédé selon l'une des revendications qui précèdent, **caractérisé par le fait que** l'agent modifiant les propriétés de surface est un agent tensio-actif.

5. Procédé selon l'une des revendications 1 à 3, **caractérisé par le fait que** l'agent modifiant les propriétés de surface est un composé lipophile choisi parmi les glycérides, les cires, les acides gras, les alcohols gras, les paraffines, les silicones et les phosphatides.

6. Procédé selon l'une des revendications 1 à 3, **caractérisé par le fait que** l'agent modifiant les propriétés de surface est un agent antioxydant.

7. Procédé selon l'une des revendications 1 à 3, **caractérisé par le fait que** l'agent modifiant les propriétés de surface est un colorant.

8. Procédé selon l'une des revendications 1 à 3, **caractérisé par le fait que** l'agent modifiant les propriétés de surface a un effet antistatique et/ou lubrifiant.
